(19)

Europäisches
Patentamt
European
Patent Office
Office européen
des brevets

(11) **EP 1 800 637 A1**

(12) **EUROPEAN PATENT APPLICATION**

(43) Date of publication:
**27.06.2007 Bulletin 2007/26**

(51) Int Cl.:
***A61F 9/01*** *(2006.01)*

(21) Application number: **06125851.3**

(22) Date of filing: **11.12.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **20.12.2005 US 313287**

(71) Applicant: **Alcon RefractiveHorizons, Inc.
Fort Worth, Texas 76134-2099 (US)**

(72) Inventors:
• **Curatu, Eugene O.
Orlando, FL 32820 (US)**
• **Olmstead, Richard Ty
Oviedo, FL 32765 (US)**

(74) Representative: **Hanna, Peter William Derek et al
Hanna, Moore & Curley
13 Lower Lad Lane
Dublin 2 (IE)**

(54) **Dynamic laser beam characteristic measurement system for opthalmic surgery and associated methods**

(57)    A system and method for determining and monitoring a laser ablation volume of a cornea (14) is disclosed. The method includes sampling a beam of laser shots from a pulsed treatment laser (11) as a reference portion (19). A fluence distribution of the beam reference portion is measured, and a laser beam characteristic is calculated (104) from the measured fluence distribution.

A system for determining and monitoring a laser ablation volume of a cornea includes a beamsplitter (16) that is positioned to split a beam of laser shots from a pulsed treatment laser into a corneal portion (18) and a reference portion (19). Devices (21) are provided for measuring a fluence distribution of the beam reference portion and for calculating an ablation volume per laser shot from the measured fluence distribution.

## Fig. 1A

EP 1 800 637 A1

## Fig. 1B

**Description**

**Field of the Invention**

The present invention is directed to laser surgery on the eye, and, more particularly, to systems and methods for calculating and monitoring a characteristic of a laser beam in real time of a laser ophthalmic surgical device.

**Background of the Invention**

In surgery on the eye, the laser system must be calibrated carefully. For example, in refractive laser surgery, typically using an excimer laser system, the volume of material ablated by each laser shot must be calibrated. It is currently known in the art to perform such a calibration measurement on a synthetic material such as a plastic that is selected for being analogous to the human cornea. Then the volume of material ablated per laser shot is calculated by a diameter of an ablation "crater" produced by a given number of laser shots.

The underlying theory of this method is based on a number of assumptions. (1) The interaction of the plastic material and the laser radiation is independent of the number of laser shots; (2) there is a well-known relationship between the volume per shot in the plastic material used for calibration and the volume per shot in the cornea; and (3) the volume per shot is calculated with the assumption that the excimer laser profile is Gaussian and rotationally symmetric.

Other laser system characteristics also must be calibrated. For example, in ultrafast laser systems it is important to determine any changes to the temporal response throughout a surgical procedure.

It would be beneficial to provide a more accurate system and method for performing calibration on a laser surgical system, such as one that could be used in real time during a surgical procedure.

**Summary of the Invention**

The present invention is directed to a system and method for determining and monitoring a laser ablation volume of a cornea. The method comprises the step of sampling a beam of laser shots from a pulsed treatment laser as a reference portion. A fluence distribution of the beam reference portion is measured, and an ablation volume per laser shot is calculated from the measured fluence distribution. *Fluence* is defined as the total amount of energy applied per unit area, measured, for example, in joules/cm$^2$.

A system for determining and monitoring a laser ablation volume of a cornea comprises a beam sampler such as a beamsplitter that is positioned to split a beam of laser shots from a pulsed treatment laser into an incident portion, for example, for effecting a treatment, and a sampling, or reference, portion. Means are provided for measuring a fluence distribution of the beam sampling portion and for calculating an ablation volume per laser shot from the measured fluence distribution.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

**Brief Description of the Drawing**

FIG. 1A is a system schematic of the ablation and monitoring system of the present invention configured for calibration.

FIG. 1B is a system schematic of the ablation and monitoring system of the present invention configured for surgery.

FIG. 2 is a flowchart of an exemplary embodiment of the method of the present invention.

**Detailed Description of the Preferred Embodiments**

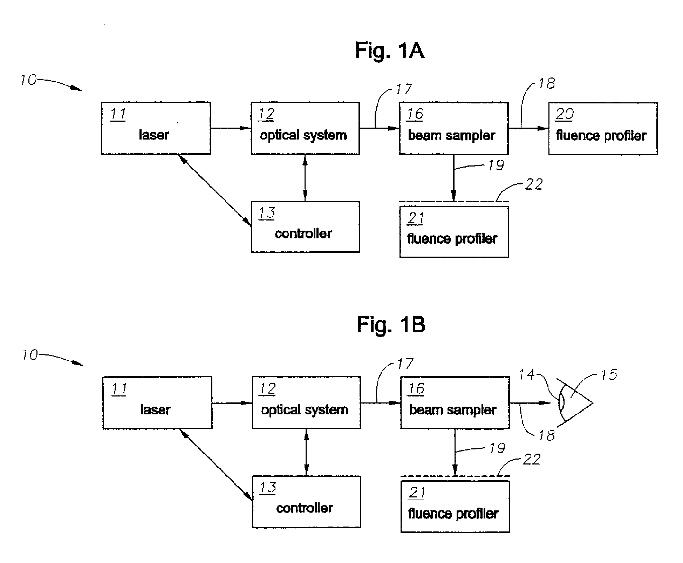

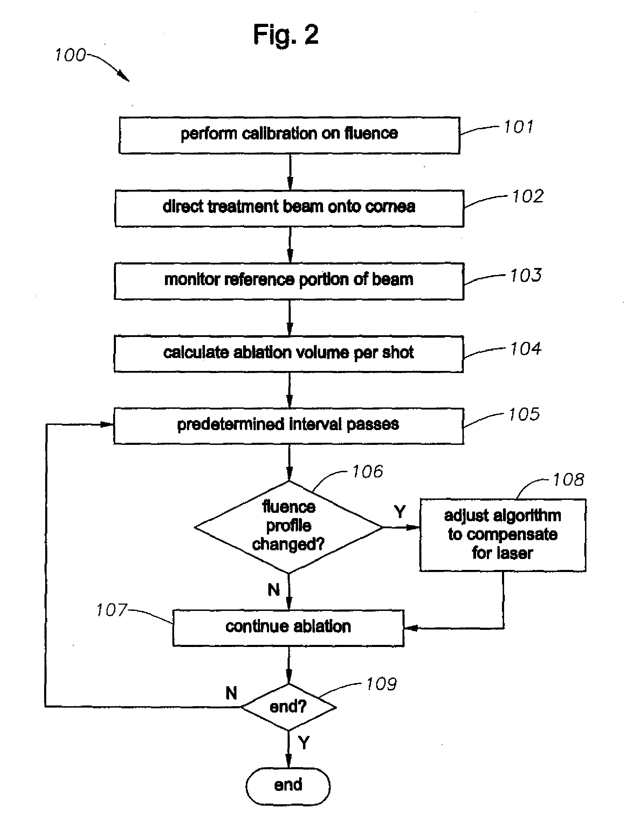

A description of preferred embodiments of the invention will now be presented with reference to FIGS. 1A-2. A system **10** for performing a corneal laser ablation comprises a pulsed treatment laser **11** that can be, for example, an excimer laser (FIGS. 1A,1B). An optical system **12** is in an optical path of the laser **11**. A controller **13** is in signal communication with the optical system **12** for controlling a placement of shots from the treatment laser **11** based upon a predetermined ablation profile. As is known in the art, the ablation profile will typically have been determined previously on a cornea **14** of an eye **15**.

A beam sampler such as a beamsplitter **16** is positioned to sample laser shots **17** from the treatment laser **11** into a treatment portion **18** and a reference portion **19**. In an exemplary embodiment for corneal refractive surgery, for example, the treatment portion **18** will be incident on the cornea **14,** and will be referred to as a "corneal portion." In a method **100** of the present invention, preferably, a calibration is performed prior to the surgery by determining a fluence distribution of the corneal portion **18** (block **101**; FIG. 1A), for example, with the use of a measurement device **20** such as a laser beam fluence profiler or energy meter. The method **100** will be described relative to the exemplary embodiment of corneal refractive surgery, but this is not intended as a limitation on the uses of the invention for other types of laser surgery.

Means for measuring a fluence distribution of the beam reference portion, for example, a laser beam fluence profiler **21,** is positioned at a corneal plane **22**. During surgery, the beamsplitter **16** directs the corneal portion **18** onto the cornea **14** in a predetermined pattern to achieve the predetermined ablation profile, under direction of the controller **13** (block **102**). The ablation profile, however, is based upon assumptions on the volume of corneal tissue ablated per shot (VPS), and it is therefore believed beneficial to provide an accurate and periodic, real-time calibration of this value.

In order to provide such a calibration, the reference portion **19** is monitored by the laser beam fluence profiler **21** (block **103**) with the use of the following calculations. If the radiance fluence or radiant exposure $F_0$ in a particular point of an excimer laser beam spot at the corneal plane, the depth of ablation per pulse can be calculated using Beer's law, which describes the attenuation of light in an optically absorbing medium:

$$F = F_0 \cdot \exp[-\alpha \cdot z] \quad (1)$$

where $F_0$ is the fluence at the separation surface, $\alpha$ is the absorption coefficient, and $F$ is the fluence at a depth $z$ in the absorbent medium.

Assuming that the ablation threshold fluence is known and the laser fluence at a point $(x,y)$ of the ablation area $A$, the ablation depth at this point is given by:

$$D(x,y) = \frac{1}{\alpha} \ln \frac{F(x,y)}{F_{TH}} \quad (2)$$

The ablation volume per laser pulse, or volume per shot VPS, is given by:

$$VPS = \iint_A D(x,y) \cdot dxdy = \frac{1}{a} \left[ \iint_A \ln F(x,y) \cdot dxdy \right] - \frac{A \cdot \ln F_{TH}}{\alpha} \quad (3)$$

and $A$ is the pulse ablation area that can be defined by $F(x,y) = F_{TH}$.

Therefore, a measurement of the fluence distribution of the pulsed excimer laser **11** at the corneal plane **22,** and applying Eq. (3) (block **104**), an accurate estimate of the volume per shot is obtained.

Preferably the reference calculation is performed at predetermined intervals during a corneal ablation procedure (block **105**). If the VPS value is substantially unchanged from the previous measurement (block **106**), no change to the ablation profile is made, and the procedure continues (block **107**). If the VPS value is changed substantially (block **106**), the system is adjusted (block **108**) prior to continuing the procedure (block **107**). This process continues until the profile is complete (block **109**).

Thus is can be seen that a more accurate and real-time calibration can be performed on the volume per shot, without making an assumption as to the beam profile, and also without making an assumption as to the equivalence between the cornea and a synthetic material.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiments thereof, and the advantageous new and useful results obtained thereby, the new and useful constructions, and reasonable mechanical equivalents thereof obvious to those skilled in the art, are set forth in the appended claims.

**Claims**

1. A method for determining and monitoring a laser beam characteristic of an ophthalmic treatment laser comprising the steps of:

   sampling a beam of laser shots from a pulsed treatment laser as a reference portion;
   measuring a fluence distribution of the beam reference portion; and
   calculating (104) a laser beam characteristic from the measured fluence distribution.

2. The method recited in Claim 1, wherein the fluence distribution is measured at a treatment plane.

3. The method recited in Claim 1, wherein the laser beam characteristic comprises an ablation volume per shot calculated as a function of an ablation depth at each point of an ablation area covered by the laser shot.

4. The method recited in Claim 3, wherein the ablation volume per shot is calculated (104) by performing an integration over the ablation area of the ablation depth, wherein the ablation depth is calculated as:

$$D(x,y) = \frac{1}{\alpha} \ln \frac{F(x,y)}{F_{TH}}$$

where $\alpha$ is the absorption coefficient, $F$ is the fluence at a point $(x,y)$, and $F_{TH}$ is the threshold fluence for a cornea (14).

5. The method recited in Claim 4, wherein the integration over the ablation area of the ablation depth equals:

$$VPS = \int\int_A D(x,y).\,dxdy = \frac{1}{a}\left[\int_a\int_A \ln F(x,y).\,dxdy\right] - \frac{A\cdot\ln F_{TH}}{\alpha}$$

where $A$ is the pulse ablation area defined by $F(x,y) = F_{TH}$, the threshold fluence for a cornea (14).

6. The method recited in Claim 1, wherein the measuring step comprises using a laser beam fluence profiler (21).

7. The method recited in Claim1, wherein the sampling step comprises splitting the beam of laser shots into a treatment portion and the reference portion, and further comprising the step of determining a fluence distribution of the treatment portion for calibrating (101) the fluence distribution of the beam reference portion measuring step.

8. The method recited in Claim 1, further comprising repeating the measuring and calculating steps at predetermined intervals (105) during an ophthalmic surgical procedure.

9. The method recited in Claim 8, further comprising the step of adjusting (108) at the predetermined intervals a treatment protocol being implemented during the procedure based upon the calculated laser beam characteristic.

10. A system for determining and monitoring a laser beam characteristic comprising:

   a sampler (16) positioned to sample a beam of laser shots from a pulsed treatment laser (11) as a reference portion (19);
   means (21) for measuring a fluence distribution of the beam reference portion; and
   means for calculating (104) a laser beam characteristic from the measured fluence distribution.

11. The system recited in Claim 10, wherein the fluence distribution measuring means (21) is positioned at a treatment plane (22).

12. The system recited in Claim 10, wherein the laser beam characteristic comprises an ablation volume per shot calculated (104) as a function of an ablation depth at each point of an ablation area covered by the laser shot.

13. The system recited in Claim 12, wherein the ablation volume per shot is calculated by performing an integration over the ablation area of the ablation depth, wherein the ablation depth is calculated as:

$$D(x,y) = \frac{1}{\alpha} \ln \frac{F(x,y)}{F_{TH}}$$

where $\alpha$ is the absorption coefficient, $F$ is the fluence at a point $F(x,y)$, and $F_{TH}$ is the threshold fluence for a cornea (14).

14. The system recited in Claim 13, wherein the integration over the ablation area of the ablation depth equals:

$$VPS = \int\int_A D(x,y).\,dxdy = \frac{1}{} \left[ \int_a \int_A \ln F(x,y).\,dxdy \right] - A \cdot \ln F_{TH}$$

where $A$ is the pulse ablation area defined by $F(x,y) = F_{TH}$, the threshold fluence for a cornea (14).

15. The system recited in Claim 10, wherein the measuring means (21) comprises a laser beam fluence profiler.

16. The system recited in Claim 10, wherein the sampler (16) comprises a beamsplitter for splitting the beam of laser shots into a treatment portion (18) and the reference portion (19), and further comprising means (20) for determining a fluence distribution of the treatment portion for calibrating (101) the fluence distribution of the beam reference portion fluence measuring means.

17. The system recited in Claim 10, further comprising a controller (13) for signaling the measuring and calculating means to perform at predetermined intervals during an ophthalmic surgical procedure.

18. The system recited in Claim 17, further comprising means for adjusting (108) at the predetermined intervals a treatment protocol being implemented during the procedure based upon the calculated laser beam characteristic.

19. A system for performing an ophthalmic surgical procedure comprising:

   a pulsed treatment laser (11);
   an optical system (12) in an optical path of the laser;
   a controller (13) in signal communication with the optical system for controlling a laser beam characteristic based upon a predetermined treatment protocol;
   a sampler (16) positioned to sample a beam of laser shots from the treatment laser as a reference portion;
   means (21) for measuring a fluence distribution of the beam reference portion (19);
   means for calculating (104) laser beam characteristic from the measured fluence distribution;
   means in communication with the controller for adjusting (108) the treatment protocol based upon the calculated laser beam characteristic.

20. The system recited in Claim 21, wherein the fluence distribution measuring means (21) is positioned at a treatment plane (22).

21. The system recited in Claim 19, wherein the laser beam characteristic comprises an ablation volume per shot calculated (104) as a function of an ablation depth at each point of an ablation area covered by the laser shot.

22. The system recited in Claim 21, wherein the ablation volume per shot is calculated (104) by performing an integration over the ablation area of the ablation depth wherein the ablation depth is calculated as:

$$D(x,y) = \frac{1}{\alpha} \ln \frac{F(x,y)}{F_{TH}}$$

where a is the absorption coefficient, $F$ is the fluence at a point $F(x,y)$, and $F_{TH}$ is the threshold fluence for a cornea (14).

23. The system recited in claim 22, wherein the integration over the ablation area of the ablation depth equals:

$$VPS = \int\int_A D(x,y).\,dxdy = \frac{1}{a}\left[\int\int_A \ln F(x,y).\,dxdy\right] - \frac{A\cdot\ln F_{TH}}{\alpha}$$

where A is the pulse ablation area defined by $F(x,y) = F_{TH}$, the threshold fluence for a cornea.

24. The system recited in Claim 19, wherein the measuring means comprises a laser beam fluence profiler (21).

25. The system recited in Claim 19, wherein the sampler comprises a beamsplitter (16) for splitting the beam of laser shots into a treatment portion (18) and the reference portion (19), and further comprising means for determining a fluence distribution of the treatment portion for calibrating (101) the fluence distribution of the beam reference portion fluence measuring means.

26. The system recited in Claim 19, further comprising a controller (13) for signaling the measuring and calculating means to perform at predetermined intervals during an ophthalmic surgical procedure.

27. The system recited in Claim 26, wherein the adjusting means operates at the predetermined intervals to adjust (108) the treatment protocol being implemented during the procedure based upon the calculated laser beam characteristic.

## Fig. 1A

10

| 11 laser | → | 12 optical system | → | 16 beam sampler | → | 20 fluence profiler |

17

18

| 13 controller |

19

22

| 21 fluence profiler |

## Fig. 1B

10

| 11 laser | → | 12 optical system | → | 16 beam sampler |

17

14  15

18

| 13 controller |

19

22

| 21 fluence profiler |

# Fig. 2

100

```
┌─────────────────────────────────┐
│   perform calibration on fluence │──── 101
└─────────────────────────────────┘
                 │
                 ▼
┌─────────────────────────────────┐
│   direct treatment beam onto cornea │──── 102
└─────────────────────────────────┘
                 │
                 ▼
┌─────────────────────────────────┐
│   monitor reference portion of beam │──── 103
└─────────────────────────────────┘
                 │
                 ▼
┌─────────────────────────────────┐
│   calculate ablation volume per shot │──── 104
└─────────────────────────────────┘
                 │
                 ▼
┌─────────────────────────────────┐
│   predetermined interval passes │──── 105
└─────────────────────────────────┘
                 │
                 ▼
              106
           ╱ fluence ╲        Y      ┌──────────────────┐
          ╱  profile   ╲──────────────│ adjust algorithm │──── 108
          ╲  changed?  ╱              │ to compensate    │
           ╲         ╱                │ for laser        │
                 │ N                  └──────────────────┘
                 ▼
107 ─┌─────────────────────┐
     │   continue ablation │◄─────────────────┘
     └─────────────────────┘
                 │
                 ▼
         N    ╱ end? ╲ ──── 109
     ┌───────╲      ╱
     │         ╲  ╱
     │          │ Y
     │          ▼
     │      ( end )
     └──────────┘ (to 105)
```

European Patent
Office

**EUROPEAN SEARCH REPORT**

Application Number

EP 06 12 5851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 928 221 A (SASNETT MICHAEL W [US] ET AL) 27 July 1999 (1999-07-27) | 1-18 | INV. A61F9/01 |
| Y | * abstract * * column 5, line 36 - column 7, line 13 * | 20 | |
| X | US 2001/003154 A1 (O'DONNELL FRANCIS E [US] O'DONNELL JR FRANCIS E [US]) 7 June 2001 (2001-06-07) * abstract * * figure 1 * | 1,3-10, 12-19, 21-27 | |
| Y | * paragraphs [0025] - [0029], [0032] - [0034] * | 20 | |
| X | EP 0 274 205 A2 (TAUNTON TECH INC [US] VISX INC [US]) 13 July 1988 (1988-07-13) * abstract * * figures 1,8 * * column 5, line 42 - line 53 * * column 10, line 1 - line 26 * | 1,8-10, 15,17 | |
| X | WO 2005/011544 A (CARL ZEISS MEDITEC AG [DE]; DICK MANFRED [DE]; VOGELSANG HARTMUT [DE]) 10 February 2005 (2005-02-10) * page 3, line 37 - page 4, line 31 * * page 5, line 12 - line 27 * | 1,2 | TECHNICAL FIELDS SEARCHED (IPC) A61F |
| A | WO 01/20277 A (VISX INC [US]; YEE KINGMAN [US]; CLAPHAM TERRANCE N [US]) 22 March 2001 (2001-03-22) * the whole document * | 1-27 | |
| A | DE 41 37 646 A1 (ZEISS CARL FA [DE]) 19 May 1993 (1993-05-19) * the whole document * | 1-27 | |

The present search report has been drawn up for all claims

| Place of search | Date of completion of the search | Examiner |
|---|---|---|
| Munich | 6 March 2007 | Jansen, Birte |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 12 5851

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2007

| Patent document cited in search report | | Publication date | Patent family member(s) | | Publication date |
|---|---|---|---|---|---|
| US 5928221 | A | 27-07-1999 | US | 6287299 B1 | 11-09-2001 |
| US 2001003154 | A1 | 07-06-2001 | NONE | | |
| EP 0274205 | A2 | 13-07-1988 | CA | 1288481 C | 03-09-1991 |
| | | | DE | 3785568 D1 | 27-05-1993 |
| | | | DE | 3785568 T2 | 28-10-1993 |
| | | | JP | 1684306 C | 31-07-1992 |
| | | | JP | 3044534 B | 08-07-1991 |
| | | | JP | 63150069 A | 22-06-1988 |
| | | | US | 4911711 A | 27-03-1990 |
| WO 2005011544 | A | 10-02-2005 | DE | 10333562 A1 | 17-02-2005 |
| WO 0120277 | A | 22-03-2001 | AU | 7377800 A | 17-04-2001 |
| | | | BR | 0013982 A | 14-05-2002 |
| | | | CA | 2384804 A1 | 22-03-2001 |
| | | | CN | 1378642 A | 06-11-2002 |
| | | | CN | 1641326 A | 20-07-2005 |
| | | | EP | 1224442 A1 | 24-07-2002 |
| | | | JP | 2003509681 T | 11-03-2003 |
| | | | MX | PA02002503 A | 30-07-2002 |
| | | | US | 2002198515 A1 | 26-12-2002 |
| | | | US | 2003149426 A1 | 07-08-2003 |
| | | | US | 6559934 B1 | 06-05-2003 |
| DE 4137646 | A1 | 19-05-1993 | NONE | | |

EPO FORM P0459

For more details about this annex : see Official Journal of the European Patent Office, No. 12/82